Europäisches Patentamt

European Patent Office

Office européen des brevets

(19)

(11) Publication number: **0 147 020**

A2

(12) **EUROPEAN PATENT APPLICATION**

(21) Application number: 84307079.8

(22) Date of filing: 16.10.84

(51) Int. Cl.⁴: **B 01 J 21/12**
B 01 J 23/10, C 07 C 1/20

(30) Priority: 09.12.83 GB 8332950

(43) Date of publication of application:
03.07.85 Bulletin 85/27

(84) Designated Contracting States:
BE DE FR IT NL

(71) Applicant: Coal Industry (Patents) Limited
Hobart House Grosvenor Place
London SW1X 7AE(GB)

(72) Inventor: Robinson, Joseph Gordon
"Claybrook" The Hyde
Winchcombe Gloucestershire(GB)

(72) Inventor: Riemer, Pierce William Foster
27 Lambourne Avenue
Huntly Gloucestershire(GB)

(74) Representative: Wood, John Irwin
Hobart House Grosvenor Place
London SW1X 7AE(GB)

(54) Improvements in catalysts.

(57) A mesoporous amorphous catalyst having a majority of pores in th size range 2-10mn and being composed of an aluminium compound chemically bonded to an amorphous silica, shows conversion of oxygen-containing aliphatic compounds into hydrocarbons with surprising selectivity to aromatics.

EP 0 147 020 A2

1

Case 4648

"IMPROVEMENTS IN CATALYSTS"

This invention concerns improvements in catalysts, more specifically it concerns mesoporous catalysts based on amorphous silica xerogel.

We have described in British Published Patent Application No. 2,100,710A, microporous alumino-silicate catalysts of a novel type, in which an aluminium compound is deposited on the surface of an amorphous silica in an amount equivalent to a monolayer covering up to 90% of the BET surface area of the silica, and is reacted with the silica. The catalysts have a maximum pore diameter of 1.5nm and are effective in the production of $C_1$-$C_7$ alkyl-benzenes from oxygen-containing aliphatics such as methanol or dimethyl ether. It has been suggested that the production of hydrocarbons from oxygen-containing aliphatics using zeolite catalysts such as the Mobil ZSM-5 catalyst is largely or wholly governed by the size of the pores in the zeolite. The hydrocarbons produced from ZSM-5 are almost exclusively in the $C_1$-$C_{10}$ range, and this is certainly consistent with steric constraints on molecular growth set by the size of the pores (0.6nm) and the free space at pore intersections (0.9nm). A similar explanation appears to fit the maximum molecular size ($C_{13}$) obtained using the

microporous alumino-silicate catalysts mentioned above, in which the examples tested most frequently had a maximum pore size of 1.0nm.

We have now surprisingly found that certain porous catalysts having essentially no pores in the micropore ($<$2nm) range and a majority of pores in the mesopore range (2-10nm) are also active in the production of hydrocarbons, especially aromatics, up to $C_{12}$, but contrary to expectations do not catalyse the formation of higher hydrocarbons.

The invention provides a mesoporous synthetic catalyst comprising a layer of an aluminium compound chemically bonded to the surface of an amorphous silica in an amount corresponding to a monolayer covering 25 to 125% of the BET surface of the silica, the catalyst having a majority of pores in the size range 2-10nm, having substantially only protons and/or cations selected from rare earth metals, alkaline earth metals and first row transition metals as charge balancing species.

The invention also provides a process for the production of the catalysts of the invention, comprising treating a dry mesoporous amorphous silica with an anhydrous solution of a hydrolysable aluminium compound, in an amount corresponding to a monolayer covering 25 to 125% of the BET surface of the silica and causing the aluminium compound to react with the surface of the silica by heating, to yield the catalyst having a majority of pores in the size range 2-10nm, displacing substantially all charge balancing species by protons and optionally ion-exchanging to deposit cations selected from rare earth metals, alkaline earth metals and first row transition metals.

Preferably, the coverage of aluminium compound corresponds to about 50 to 110% monolayer coverage, especially about 100%. This is in contrast to the disclosure in Specification No. 2,100,710A, in which a maximum of 90% coverage is specified; and in which coverage of about 50% was preferred. Nonetheless, the catalysts may be prepared by methods based on those disclosed in said Specification and in our co-pending United Kingdom Application No. 2,132,595A, although a mesoporous silica starting material is required to ensure the production of the catalysts of the present invention. Suitable mesoporous silica xerogels are commercially available and preferably have substantially no pores in the size range $<2$nm and preferably almost all the pores (based on pore volume measurements) in the size range 2-5nm. It was found in tests that although the process of the invention resulted in a loss of pore volume, there was substantially no production of micropores.

Suitably, the starting material, silica xerogel, is treated with a solution of the predetermined amount of an aluminium compound, which is suitably an aluminium alkoxide, in an anhydrous solvent such as an alkane, removing the solvent to leave a layer of the compound on the surface of the silica. The compound is caused to react with the surface of the silica by heating, suitably to a temperature of from 400 to 900°C, preferably about 450 to 550°C, especially about 500°C. It is preferred not to exceed a temperature of about 1200°C at any time in the handling or preparation of the present catalysts.

Preferably, the silica xerogel having the layer of reacted aluminium compound is treated with an ammonium salt solution capable of exchanging labile cations for ammonium ions, washing the treated material with deionised water until no further ammonium cations can be detected and drying and calcining the material at a temperature of from 250 to 1200°C to yield the catalyst having substantially only protons as surface charge balancing species. Preferably, the temperature of calcination is 250 to 900°C, more preferably 450 to 650°C, and suitably is carried out for 4 to 10 hours.

An ion-exchange step may be incorporated. Such ion exchange steps have been described in the literature dealing with crystalline zeolites, and analogous methods may be used, suitably using a salt of the selected metal, such as a nitrate. The cations are preferably selected from the rare-earth metals and alkaline earth metals.

The catalyst of the invention is especially useful in the conversion of oxygen-containing aliphatics to hydrocarbons and water, such oxygen-containing aliphatics being preferably selected from alcohols and ethers and especially being methanol and/or dimethyl ether. The catalyst may be used alone or in combination with, that is admixed with or directly subsequent to, a catalyst of a different type, for example a Fischer-Tropsch type catalyst for the direct conversion of synthesis gas.

The invention also provides, therefore, a process for the conversion of an oxygen-containing aliphatic compound into $C_1-C_{12}$ hydrocarbons, comprising passing the oxygen-containing aliphatic compound in the vapour form over the catalyst of the invention, at a temperature of 250 to 600°C. The process may be carried out at ambient, or a higher or lower pressure.

The invention will now be described by way of example only.

EXAMPLE

100g of silica xerogel (A-quality, grade SD210 supplied by Crosfield Ltd., England) was dried by heating at 120°C for 4h. The dry xerogel was then cooled in a desiccator, transferred to a dry flask and covered with 250ml of a 14.05% w/w solution of aluminium sec-butoxide in dry n-hexane. This was the estimated concentration needed to cover 25% of the BET surface of xerogel with a monolayer of the aluminium butoxide after removal of the excess solution. This procedure was repeated with samples of the xerogel being soaked in 28.1%, 42.1%, 56.2% and 70.2% w/w solutions, so as to give to 50%, 75%, 100% and 125% respectively of monolayer coverage.

The pressure in the flask was reduced and after 12h the excess liquor was decanted. The product was transferred to a vacuum oven and the pressure was reduced over a 3h period and then the temperature was raised to 60°C for 1h, to remove residual solvent. After cooling the product, it was soaked in deionised water, the pressure was reduced and the flask left for 16h at room temperature. After drying, the product was heated at 500°C for 4h to complete reaction of the aluminium compound with the xerogel.

A 25g sample of the above prepared product was packed into a stainless steel tube and a 0.1M aqueous solution of ammonium nitrate was passed through to exchange labile cations for ammonium ions. Samples of the eluent were tested for the presence of aluminium periodically, and when none could be detected, the ammonium nitrate solution was displaced by deionised water and this was passed until the pH of the eluent was identical to that of the feed water. The product was removed from the column, dried then calcined at 600°C for 6 hours to yield a catalyst in which substantially only protons were present as charge balancing species.

It has now been found that if a 1.0 molar solution of ammonium nitrate, at 80°C, is used instead of the 0.1M solution at room temperature mentioned above, an appreciable saving in time in the method can be made, without significantly altering the resultant catalyst or its activity.

Conventional adsorption/desorption of nitrogen tests on the starting material xerogel and the final catalyst gave results on surface areas and pore size disbribution as shown in Table 1 below.

TABLE 1

| Product | BET surface m 2/g | Pore volume (mm3/g) for different pore width ranges | | | |
|---|---|---|---|---|---|
| | | 0-2nm | 2-5nm | 5-10nm | 10-60nm |
| xerogel | 509 | 0 | 778 | 49 | 17 |
| 25% monolayer cover catalyst | 415 | 0 | 686 | 21 | 11 |
| 100% monolayer cover catalyst | 355 | 0 | 578 | 20 | 11 |

The catalysts prepared were tested for the conversion of methanol by pumping methanol at a LHSV of 1.0 $h^{-1}$ through a pre-heater packed with glass beads which was held at a temperature of 5°C below that of the catalyst which was packed into a fixed bed in a tubular reactor. The conversion at various bed temperatures was assessed and the products collected and analysed. The compositon of the products in wt%are shown in Table 2 below.

Viewing the results, it was surprising that there was no production of hydrocarbons greater than $C_{12}$, and also that there was relatively little production of toluene. The pore spaces available could not of themselves have restricted the size range of molecules to that observed, and some other mechanism must have been involved. That is, the catalysts of the present invention demonstrate an unexpected size selectivity.

Table 2

| Monolayer coverage of the BET surface area (%) | 25 | | | | | 50 | | | | | 75 | | | | | 100 | | | | | 125 | | | | |
|---|---|---|---|---|---|---|---|---|---|---|---|---|---|---|---|---|---|---|---|---|---|---|---|---|---|
| Catalyst bed temp. (°C) | 250 | 300 | 350 | 400 | 450 | 250 | 300 | 350 | 400 | 450 | 250 | 300 | 350 | 400 | 450 | 250 | 300 | 350 | 400 | 450 | 250 | 300 | 350 | 400 | 450 |
| Methane | | 43.0 | 31.1 | 23.5 | 26.0 | | 20.3 | 20.7 | 28.5 | 18.9 | | 15.7 | 16.9 | 17.4 | 14.9 | 8.6 | 4.1 | 6.4 | 6.4 | 8.5 | 4.9 | 8.0 | 9.6 | 9.8 | 9.2 |
| Ethane | | 0.8 | 0.5 | 0.4 | 0.4 | | 0.4 | 0.5 | 0.5 | 0.4 | | 0.5 | 0.4 | 0.5 | 0.4 | 0.3 | 0.2 | 0.2 | 0.3 | 0.2 | 0.4 | 0.4 | 0.6 | 0.6 | 0.6 |
| Ethene | | 1.6 | 1.3 | 0.8 | 0.9 | | 1.4 | 1.0 | 1.3 | 1.2 | | 2.3 | 2.1 | 2.0 | 2.3 | 1.4 | 0.9 | 0.9 | 1.1 | 0.7 | 1.1 | 2.1 | 1.7 | 2.4 | 2.3 |
| Propane | | 0.1 | 0.1 | – | – | | – | – | – | – | | – | 0.1 | 0.1 | 0.1 | 0.1 | – | – | 0.1 | 0.1 | 0.1 | 0.1 | 0.1 | 0.1 | 0.1 |
| Propene | | 0.8 | 0.7 | 0.4 | 0.5 | | 0.5 | 0.5 | 0.6 | 0.4 | | 1.4 | 1.5 | 1.7 | 1.4 | 1.0 | 0.7 | 0.3 | 0.4 | 0.8 | 0.8 | 1.2 | 1.4 | 1.8 | 1.6 |
| Iso-Butane | | 0.1 | 0.1 | 0.1 | 0.1 | | – | – | – | – | | 0.1 | 0.1 | 0.1 | 0.1 | 0.1 | – | – | 0.1 | – | 0.1 | 0.1 | 0.1 | 0.1 | 0.1 |
| n-Butane | | – | – | – | – | | – | 0.1 | – | – | | 0.1 | 0.1 | – | – | – | – | – | – | – | – | – | – | – | – |
| Butene-1 | | 0.6 | 0.4 | 0.3 | 0.3 | | 0.4 | 0.3 | 0.3 | 0.4 | | 0.3 | 0.3 | 0.4 | 0.3 | 0.3 | 0.2 | 0.2 | 0.3 | 0.3 | 0.2 | 0.3 | 0.4 | 0.4 | 0.3 |
| Iso-Pentane | | 0.1 | – | 0.1 | – | | – | – | – | – | | 0.1 | – | – | – | 0.1 | – | – | – | – | – | 0.1 | 0.1 | – | 0.1 |
| Cyclohexane | | 0.1 | – | 0.1 | – | | – | – | 0.6 | 0.3 | | – | – | – | – | – | – | – | – | – | – | – | – | – | – |
| n-Hexane | | – | – | – | – | | – | – | 0.1 | – | | – | 0.1 | – | – | – | – | – | – | – | – | – | – | – | – |
| Methylcyclohexane | | 0.1 | 0.1 | – | – | | – | – | – | – | | – | 0.1 | – | – | – | – | – | – | – | – | – | – | – | – |
| Pentene | | 0.5 | 0.4 | 0.3 | 0.3 | | 0.3 | 0.3 | 0.4 | 0.3 | | 0.3 | 0.3 | 0.3 | 0.3 | 0.2 | 0.1 | 0.3 | 0.2 | 0.2 | 0.2 | 0.3 | 0.3 | 0.3 | 0.3 |
| Hexene | | 0.2 | 0.1 | 0.1 | 0.1 | | 0.1 | – | 0.1 | – | | 0.1 | 0.1 | 0.1 | 0.1 | 0.1 | – | 0.1 | 0.1 | – | 0.1 | 0.1 | 0.1 | 0.1 | 0.1 |
| Toluene | | – | – | – | – | | – | – | – | – | | – | – | – | – | – | – | – | – | – | – | – | – | – | – |
| $C_2$-Alkylbenzenes | | 3.3 | 7.3 | 10.1 | 9.9 | | 8.8 | 9.6 | 7.4 | 10.7 | | 7.3 | 10.0 | 9.0 | 10.2 | 8.4 | 9.4 | 8.1 | 8.4 | 9.5 | 11.2 | 7.4 | 6.4 | 6.2 | 5.1 |
| $C_3$-Alkylbenzenes | | 6.6 | 12.6 | 16.9 | 16.8 | | 14.8 | 15.2 | 12.3 | 16.9 | | 11.7 | 11.6 | 9.8 | 11.4 | 13.4 | 17.8 | 14.5 | 13.4 | 14.2 | 13.8 | 10.8 | 10.1 | 8.7 | 9.8 |
| $C_4$-Alkylbenzenes | | 6.2 | 13.3 | 18.1 | 15.5 | | 16.9 | 17.0 | 13.7 | 17.5 | | 15.3 | 14.5 | 13.3 | 15.1 | 19.7 | 24.3 | 21.4 | 19.0 | 20.7 | 14.5 | 12.8 | 11.6 | 11.0 | 12.9 |
| $C_5$-Alkylbenzenes | | 5.6 | 10.1 | 12.8 | 13.7 | | 12.9 | 15.0 | 10.0 | 13.5 | | 14.5 | 11.3 | 13.1 | 14.9 | 18.4 | 23.5 | 23.2 | 20.2 | 21.4 | 22.0 | 14.6 | 13.4 | 10.2 | 11.7 |
| $C_6$-Alkylbenzenes | | 1.1 | 2.7 | 3.0 | 2.2 | | 3.2 | 2.4 | 2.5 | 4.3 | | 2.5 | 3.0 | 2.5 | 2.2 | 1.9 | 3.1 | 2.1 | 6.8 | 2.9 | 3.7 | 3.3 | 1.8 | 2.3 | 3.1 |
| $C_7$-Alkylbenzenes | | – | – | – | – | | – | – | – | – | | – | – | – | – | – | – | – | – | – | – | – | – | – | – |
| Carbon monoxide | | 2.9 | 1.7 | 1.2 | 1.3 | | 1.9 | 2.3 | 3.8 | 1.0 | | 1.6 | 2.2 | 2.1 | 1.8 | 0.9 | 0.7 | 0.6 | 0.7 | 0.9 | 1.2 | 3.1 | 3.1 | 3.5 | 3.5 |
| Carbon dioxide | | 0.7 | 0.6 | 0.5 | 0.5 | | 0.8 | 0.5 | 0.9 | 0.6 | | 0.4 | 0.5 | 0.6 | 0.6 | 0.2 | 0.1 | 0.2 | 0.3 | 0.2 | 0.3 | 0.5 | 0.5 | 0.5 | 0.5 |
| Dimethyl ether | | 25.8 | 16.8 | 11.5 | 11.4 | | 16.9 | 14.6 | 16.8 | 13.4 | | 25.9 | 24.9 | 27.1 | 24.0 | 24.7 | 14.6 | 21.1 | 22.1 | 19.5 | 25.5 | 36.2 | 38.8 | 41.9 | 38.5 |
| Methanol conversion % | 0 | 5.8 | 16.2 | 21.5 | 23.7 | 0 | 16.0 | 21.8 | 35.2 | 34.9 | 1.5 | 30.7 | 35.6 | 40.0 | 41.2 | 7.0 | 39.8 | 71.8 | 84.7 | 93.5 | 10.1 | 32.8 | 47.8 | 52.1 | 55.2 |

Y8346PA264 (WPX1)

Case 4648/EURO

PATENT CLAIMS:

1.   A mesoporous synthetic catalyst characterised in that a layer of an aluminium compound is chemically bonded to the surface of an amorphous silica in an amount corresponding to a monolayer covering 25 to 125% of the BET surface of the silica, and further characterised in that the catalyst has a majority of pores in the size range 2-10mn, and has substantially only protons and/or cations selected from rare earth metals, alkaline earth metals and first row transition metals as charge balancing species.

2.   A catalyst according to claim 1, characterised in that the majority of pores are in the size range 2-5mn.

3.   A catalyst according to claim 1 or 2, characterised in that the coverage of aluminium compound corresponds to 50 to 110% monolayer coverage.

4.   A catalyst according to any one of claims 1 to 3, characterised in that the charge balancing species are essentially only protons.

5.   A process for the production of catalysts according to claim 1, characterised in that (a) a dry mesoporous amorphous silica is tested with an anhydrous solution of a hydrolysable aluminium compound, in an amount corresponding to a monolayer covering 25 to 125% of the BET surface of the silica, (b) the aluminium compound is caused to react with the surface of the silica by heating, to yield the catalyst having a majority of pores in the size range 2-10mn, (c) substantially all surface charge balancing species are displaced by protons and optionally cations selected from rare each metals, alkaline earch metals and first row transition metals are deposited by ion-exchange.

6.   A process according to claim 5, characterised in that the amount of aluminium compound corresponds to a monolayer covering 50 to 110%

- 10 -

0147020

of the BET surface of the silica.

7.    A process according to claim 5 or 6, characterised in that the aluminium compound is caused to react with the silica by heating at a temperature of from 400 to 900$^{o}$C.

8.    A process according to any one of claims 5 to 7, characterised in that the catalyst, in the protonic form, is calcined at a temperature of 250 to 900$^{o}$C.

9.    A process for the conversion of an oxygen-containing aliphatic compound into $C_1-C_{12}$ hydrocarbons, characterised in that the compound in the vapour form is passed over a catalyst according to any one of claims 1 to 4, at a temperature of 250 to 600$^{o}$C.